# EUROPEAN PATENT APPLICATION

(11) **EP 3 842 155 A1**
(43) Date of publication of application: **30.06.2021**
(21) Application number: 20215910.9
(22) Date of filing: 21.12.2020
(51) Int. Cl.: B05B 11/00, A61Q 19/10

(54) **KIT, SYSTEM AND CLEANING COMPOSITION FOR PERSONAL HYGIENE (MOUSSE)**

(30) Priority: 23.12.2019 IT 201900025321
(71) Applicant: Madel S.p.A., 48033 Cotignola (Ravenna) (IT)
(72) Inventor: SEBASTIANI, Giacomo, 48022 LUGO (Ravenna) (IT); DELLA CUNA, Maurizio, 48022 LUGO (Ravenna) (IT)
(74) Representative: Puggioli, Tommaso

(57) **Abstract**

Described is a cleaning system for personal hygiene comprising a bottle (1) having an open neck (3), a spraying device (2), comprising a pumping unit (4), associated with said bottle at the open neck, a cleaning composition (C) inserted inside the bottle (1); the cleaning composition (C) comprises:
(A) 1-20% by weight, preferably 1-15% by weight, more preferably 1.5-10% by weight, of a mixture of surfactants comprising at least one nonionic surfactant and at least one amphoteric surfactant;
(B) water up to 100% by weight,
wherein the quantities of (A) and (B) are referred to the total weight of (A)+(B), and
said pumping unit (4) comprises a pump (41) configured for dispensing between 1 cc and 2 cc of cleaning composition (C) for a stroke of said lever (5).

## Description

This invention relates to a kit, a system and a cleaning composition for personal hygiene and in particular for washing the body.

There are many prior art products for washing the body, known as, for example, bath foam, bubble bath, shower gel and the like which, in liquid form, are used for cleaning the body.

Usually, during washing, for example in a bath or in a shower, these products are used in combination with the water and, due to their liquid nature, with a support for retaining them, such as a sponge.

The prior art bath foams or shower gels are normally packaged in closed bottles and would be immediately dispersed with the washing water once extracted from the respective container if not retained by a suitable support used in turn as a means for applying cleaning composition to the body.

One drawback of these prior art washing systems, basically consisting of sponge together with a liquid cleaning product, is a relatively high consumption of product which remains soaked into the sponge instead of being used on the body.

Moreover, as is known, the sponges used for cleaning become receptacles for bacteria which are difficult to eliminate.

Lastly, the extraction of the liquid products from the respective bottles, although simple, always requires relative attention and often implies a waste of cleaning composition.

In this context, the main aim of the invention is to overcome the above-mentioned drawbacks.

An aim of the invention is to provide a system and a cleaning composition for personal hygiene which allow a significant saving of cleaning composition.

Another aim of the invention is to provide a system and a cleaning composition for personal hygiene which allows a practical dispensing of the cleaning composition.

Another aim of the invention is to provide a system and a cleaning composition for personal hygiene which makes it possible to reduce or avoid the use of sponges during washing.

Further features and advantages of the invention are more apparent in the detailed description below, with reference to a preferred, non-limiting, embodiment of a system and a cleaning composition for personal hygiene in which:
- Figure 1 is a schematic perspective view of a bottle equipped with a spraying device with a lever forming part of the system for personal hygiene according to the invention;
- Figure 2 is a schematic cross-section view, partly in blocks, of the spraying device of Figure 1.

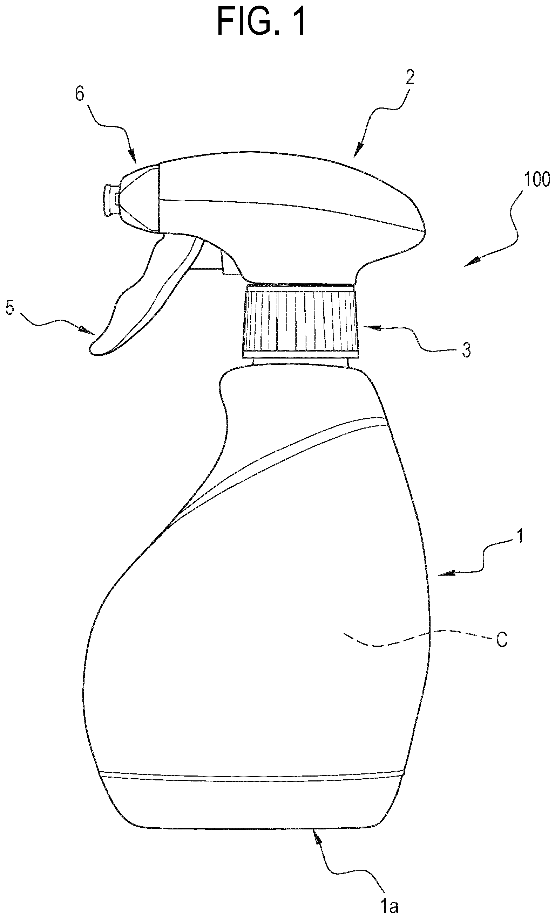

With reference to Figure 1, the numerals 1 and 2 denote, respectively, a bottle and a spraying device with a lever forming part of a cleaning system 100 according to the invention.

The cleaning system 100, as described in more detail below, also comprises a cleaning composition C for personal hygiene which when dispensed with the spraying device 2 makes it possible to obtain a soft, compact and stable mousse (or foam), which slides slowly when applied on the skin.

The term "mousse" refers in the context of this description and in the appended claims to a mixture for dispersion of particles of air in a liquid.

The bottle 1 has an open neck 3 and the spraying device 2, of substantially known type and described only insofar as necessary for understanding this solution, is associated, for example screwed, to the bottle 1 and in particular to the neck 3 of the bottle.

The spraying device 2 comprises a pumping unit 4 in fluid communication with the inside of the bottle 1 for drawing out and dispensing a liquid content prepared in the bottle 1.

According to the invention, the liquid content of the bottle 1 is the cleaning composition C for personal hygiene to which explicit reference will be made.

The spraying device 2 comprises a lever or trigger 5 for actuating the unit 4 and a diffuser unit 6 for dispensing the contents of the bottle 1 moved by the pumping unit 4 and drawn out from the bottle 1.

The lever 5 is movable between a stable rest position, illustrated for example in the accompanying drawings, in which it is away from the bottle 1, and a dispensing position, in which it is close to the bottle 1, not illustrated; a passage from the rest position to the dispensing position is also referred to as the "stroke" of the lever 5.

In known manner, an actuation of the lever 5, that is to say, a pulling of the lever into the dispensing position, corresponds to a dispensing of at least part of the contents of the bottle 1.

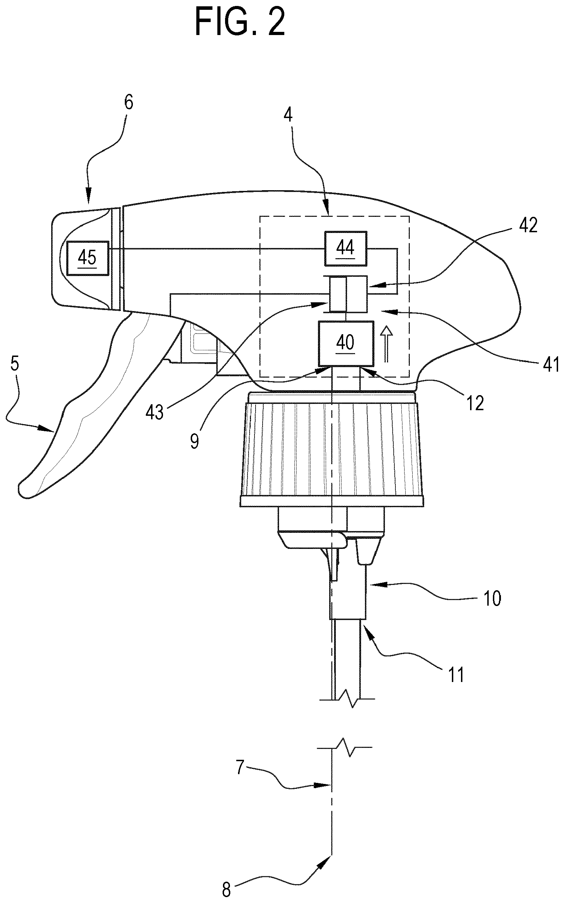

As schematically illustrated in Figure 2, the spraying device 2 comprises a conduit 7 for drawing out the contents of the bottle 1.

The conduit 7 has an inlet 8 in fluid communication with the inside of the bottle 1 through the neck 3 for drawing out the contents of the bottle 1 and an outlet 9 in communication with the pumping unit 4.

The inlet 8 of the conduit 7 is positioned, in use, substantially at a bottom 1a of the bottle 1 inside it.

Preferably, the spraying device 2 comprises a second conduit 10 having an inlet 11 in fluid communication with the inside of the bottle through the neck 3 for drawing out the contents of the bottle 1 and an outlet 12 in communication with the pumping unit 4.

The inlet 11 of the second conduit 10 is positioned substantially at the neck 3 of the bottle 1, inside it, to allow operation of the spraying device even when overturned.

Advantageously, this feature helps to make the use of the cleaning system 100 easy also, for example, under the shower during washing operations. As schematically illustrated, the conduit 10 is shorter than the conduit 7 since it has to draw out the contents of the bottle 1 in two different positions.

Schematically, the pumping unit 4 comprises a non-return valve, schematically illustrated with a block 40 in Figure 2, which receives as input the contents of the bottle 1 from the conduits 7 and 10.

The valve 40 allows the passage of the contents of the bottle 1 from the conduits 7 and 10 in the direction V towards the diffuser unit 6. Downstream of the valve 40, the pumping unit 4 comprises a pump 41 comprising a cylinder 42 in which a piston 43 slides.

The piston 43 is actuated by the lever 5 in substantially known manner not described and illustrated.

A pulling or stroke of the lever 5, opposed, for example, by a spring not illustrated, causes compression of the liquid contained in the cylinder 42; thanks to the presence of the valve 40, the liquid cannot return towards the inside of the bottle 1 through the conduits 7 and 11.

A release of the lever 5, pushed, for example, in this step, by the above-mentioned spring, causes, whilst the lever 5 returns to the rest position, a negative pressure in the cylinder 42 which returns the contents of the bottle 1 into the cylinder 42 itself.

The pumping unit 4 comprises an automatic valve, schematically illustrated with a block 44 in Figure 2, interposed between the cylinder 42 and the diffuser unit 6.

The valve 44 is configured to open when a predetermined pressure is reached at its inlet, downstream of the piston 43, that is to say, in practice, in the cylinder 42.

Preferably, the valve 44 is configured to allow the passage of the liquid contained in the bottle 1, that is, of the cleaning composition C, when the pressure of the liquid exceeds 2 bar and even more preferably when the pressure of the liquid exceeds 3 bar.

In that way, the cleaning composition C is pre-compressed at a predetermined pressure before being dispensed.

The liquid is thus dispensed at a relatively high speed and its entire volume, present in the cylinder 42, is dispensed, substantially, all at once when the lever 5 is actuated, so as to obtain the above-mentioned mousse.

Preferably, each actuation of the lever 5 dispenses between 1 cc and 2 cc of cleaning composition C, that is to say, the pump 41 dispenses between 1 cc and 2 cc of cleaning composition C for each stroke of the lever 5. Even more preferably, each actuation of the lever 5 dispenses between 1.15 cc and 1.6 cc of cleaning composition C, for example as a function of the volume of the cylinder 42 or of the stroke of the piston 43.

The quantity of cleaning composition C dispensed for each stroke of the lever 5 in combination with the shape of the spraying device 2 and with the formulation of the cleaning composition contributes to the formation of the above-mentioned mousse.

According to an embodiment, the diffuser unit 6 comprises a nozzle, schematically represented as a block 45 in Figure 2, through which the contents of the bottle 1 dispensed through the spraying device 2 pass. Preferably, the nozzle 45 allows the passage of a quantity of cleaning composition C of between 1 cc and 1.6 cc per stroke of the lever 5 and still more preferably allows the passage of a quantity of cleaning composition C of between 1.15 cc and 1.35 cc per stroke of the lever 5.

According to an embodiment, the nozzle 45 allows approximately 1.15 cc to be dispensed per stroke of the lever 5.

According to an embodiment, the nozzle 45 allows approximately 1.35 cc to be dispensed per stroke of the lever 5.

According to the invention, the cleaning system 100 comprises, as mentioned, the cleaning composition C inserted inside the bottle 1.

The cleaning composition C defines the contents of the bottle 1.

The cleaning composition C is a foamy cleaning composition for personal hygiene.

Advantageously, the bottle 1 does not contain pressurised gas and, in that way, it may be filled again with cleaning composition C once it is empty. The cleaning composition C comprises:
(A) 1-20% by weight, preferably 1-15% by weight, more preferably 1.5-10% by weight, of a mixture of surfactants comprising at least one non-ionic surfactant and at least one amphoteric surfactant;
(B) water up to 100% by weight,
wherein the quantities of (A) and (B) are referred to the total weight of (A)+(B).

According to an embodiment, the cleaning composition C comprises:
(A) 1-20% by weight, preferably 1-15% by weight, more preferably 1.5-10% by weight of a mixture of surfactants consisting of at least one anionic surfactant, at least one non-ionic surfactant and at least one amphoteric surfactant;
(B) water up to 100% by weight,
wherein the quantities of (A) and (B) are referred to the total weight of (A)+(B).

Preferably, according to the latter embodiment, the mixture of surfactants comprises a quantity greater than or equal to 65% by weight, preferably 65-90% by weight, more preferably 70-85% by weight of at least one anionic surfactant, wherein the quantity of anionic surfactant in the mixture of surfactants is referred to the total weight of the mixture (A) of surfactants.

According to an embodiment, the mixture of surfactants can consist of:
(a) 65-90% by weight, preferably 70-85% by weight, of at least one anionic surfactant;
(b) 8-20% by weight, preferably 10-17% by weight, of at least one non-ionic surfactant;
(C) 2-15% by weight, preferably 5-13% by weight, of at least one amphoteric surfactant,
wherein the quantities (a), (b) and (c) are referred to the total weight of (a)+(b)+(c).

According to the embodiments described above, the non-ionic surfactant is preferably selected in the group consisting of alkyl polyglucosides, alkyl polypentosides and mixtures thereof, wherein said polyglucosides and alkyl polypentosides are preferably obtained from vegetable sources, such as, for example, wheat, corn, rice, etc.

According to a preferred embodiment, the non-ionic surfactant is preferably a mixture of alkyl polyglucosides, more preferably a mixture of lauryl/myristyl glucosides and caprylyl glucosides derived from wheat. According to the above-mentioned embodiments, the amphoteric surfactant is preferably selected in the group consisting of cocoamphodiacetates, cocoiminodipropionate, cocoamidopropylbetaine, cocoamidopropyl-dimethyl-hydroxysulphobetaine and mixtures thereof, preferably it is disodium cocoamphodiacetate.

When present, the anionic surfactant is preferably selected in the group consisting of lauryl sulphate, lauryl ether sulphate, sulpholaurates, sulphoacetates, sulphosuccinates and mixtures thereof; preferably, the anionic surfactant is selected from sulpholaurates, sulphoacetates, sulphosuccinates and mixtures thereof, wherein more preferably said sulpholaurates, sulphoacetates and sulphosuccinates are non-ethoxylated.

According to a preferred embodiment, the anionic surfactant is a mixture of sulpholaurates and sulphoacetates, preferably non-ethoxylated, more preferably a mixture of sodium lauryl sulfoacetate, sodium methyl 2-sulfolaurate and disodium 2-sulfolaurate.

According to an embodiment, the anionic surfactant is preferably selected in the group consisting of derivatives of amino acids such as, for example, glycinates, glutamates, alaninates, sarcosinates and mixtures thereof. These anionic surfactants can give greater "body" and durability to the mousse obtained with the cleaning system 100.

According to an embodiment, the mixture of surfactants does not contain lauryl sulphate or lauryl ether sulphate, in particular does not contain sodium lauryl sulphate or sodium lauryl ether sulphate. In fact, these anionic surfactants are less preferable as they can be irritating for the skin. The cleaning composition C may also comprise at least a further component (D) selected in the group consisting of vegetable extracts, fragrances, preservatives, hydrating agents, dyes, pH regulators, sequestering agents and their mixtures. According to an embodiment, the cleaning composition C comprises a quantity less than or equal to 5.0% by weight, preferably 0.1-5.0% by weight, more preferably 1.0-4.0% by weight, of the further component (D), wherein the quantity of the further component (D) is referred to the total weight of the cleaning composition C.

According to an embodiment, the cleaning composition C can consist of the components (A), (B) and (D) described above in detail.

The cleaning composition C has at least one of the following properties:
- a pH value of approximately 5.0-6.0; and/or
- viscosity less than or equal to 100 cPs, preferably of 0-100 cPs, more preferably 0-50 cPs, more preferably 0-40 cPs, more preferably 0-15 cPs; according to an embodiment, the viscosity is approximately 4-5 cPs, the viscosity being measured with a Brookfield viscometer LV-DV2, Sp31 100RPM - 20°C.

According to an embodiment, the cleaning composition C has all the above-mentioned properties.

It has been experimentally observed that for viscosity values of the cleaning composition C greater than 100 cPs the dispensing of the composition is less comfortable since the lever 5 returns to the rest position after a lengthy time.

The cleaning composition C may be prepared using methods and apparatuses known in the sector of cosmetics and in particular cleansing and therefore not described further.

The invention also relates to a kit for preparing the cleaning system 100 described above. According to the invention, a cleaning kit for preparing a cleaning system 100 for personal hygiene comprises:
- a first component comprising the bottle 1 associated with the spraying device 2; and
- a second component comprising the cleaning composition C.

The cleaning composition C of the cleaning kit may be packaged, for example, in a corresponding bag not illustrated, from which it must be poured into the bottle 1 to obtain, during use of the cleaning system 100, the above-mentioned mousse.

Advantageously, the cleaning composition C may be packaged as a refill of the bottle 1 since, as mentioned, the cleaning system 100 does not comprise pressurised gas inside it.

The invention also relates to a method for obtaining a foam cleaner for personal hygiene.

The method for obtaining a foam cleaner for personal hygiene comprises the step (i) of dispensing the cleaning composition C by actuating, preferably manually, the lever or trigger 5 of the spraying device 2 included in the cleaning system 100.

According to an embodiment, the step (i) comprises actuating the lever 5 generating a pressure of the cleaning composition C greater than or equal to 2 bar, preferably greater than or equal to 3 bar and subsequently dispensing the cleaning composition C.

According to an embodiment, the step (i) comprises dispensing 1-2 cc of cleaning composition C per stroke of the lever 5, preferably 1.15-1.6 cc. According to an embodiment, before the step (i) the method comprises a step (0) of inserting a cleaning composition C in the bottle 1 equipped with the spraying device 2.

Another object of the invention is the use of the cleaning composition C to obtain a foam cleaner for personal hygiene, that is, a mousse, the use comprising dispensing the cleaning composition C by means of the spraying device 2 described above.

Advantageously, the cleaning system 100 makes it possible to obtain, by spraying the cleaning composition C with the spraying device 2, the mousse described above which does not need support, for example a sponge, so that it can be distributed uniformly on the body as it maintains its shape once dispensed. The mousse can be dispensed, for example, directly on a hand and applied to the body, eliminating the use of sponges and avoiding the drawbacks linked to them. In particular, there is no bacterial contamination and a significant saving of product is obtained.

## Claims

1. A cleaning system for personal hygiene, comprising
- a bottle (1) having an open neck (3);
- a spraying device (2) associated with said bottle at the open neck;
- a cleaning composition (C) inserted inside the bottle (1),
said spraying device (2) comprising a pumping unit (4) in fluid communication with the inside of the bottle (1) for drawing out said cleaning composition (C), a lever (5) for actuating the pumping unit (4), a diffuser unit (6) for dispensing the cleaning composition moved by said pumping unit (4) and drawn from said bottle (1),
said cleaning system being **characterised in that**
said cleaning composition (C) comprises:
(A) 1-20% by weight, preferably 1-15% by weight, more preferably 1.5-10% by weight, of a mixture of surfactants comprising at least one non-ionic surfactant and at least one amphoteric surfactant;
(B) water up to 100% by weight,
wherein the quantities of (A) and (B) are referred to the total weight of (A)+(B), and
said pumping unit (4) comprising a pump (41) configured for dispensing between 1 cc and 2 cc of cleaning composition (C) for a stroke of said lever (5).

2. The cleaning system according to claim 1, wherein said pump (41) is configured for dispensing between 1.15 cc and 1.6 cc of cleaning composition C for said stroke of the lever (5).

3. The cleaning system according to claim 1 or 2, wherein said pumping unit (4) comprises an automatic valve (44) interposed between the pump (41) and the diffuser unit (6) configured to allow a passage of the cleaning composition (C) from the pump (41) to the diffuser unit when the pressure of the cleaning composition (C) upstream of the automatic valve (44) exceeds 2 bar.

4. The cleaning system according to claim 3, wherein said automatic valve (44) is configured to allow a passage of the cleaning composition (C) from the pump (41) to the diffuser unit when the pressure of the cleaning composition (C) upstream of the automatic valve (44) exceeds 3 bar.

5. The cleaning system according to any one of the preceding claims, wherein said diffuser unit (6) comprises a nozzle (45) through which the contents of the bottle dispensed through the spraying device (2) pass to the outside of the spraying device (2), said nozzle (45) being configured to allow a passage of a quantity of cleaning composition (C) of between 1 cc and 1.6 cc for a stroke of said lever (5).

6. The cleaning system according to claim 5, wherein said nozzle (45) is configured to allow a passage of a quantity of cleaning composition (C) of between 1.15 cc and 1.35 cc for a stroke of said lever (5).

7. The cleaning system according to any one of the preceding claims, wherein there is no gas under pressure inside said bottle (1).

8. The cleaning system according to any one of the preceding claims, wherein said spraying device (2) comprises a first conduit (7) having an inlet (8) in fluid communication with the inside of the bottle (1) through the open neck (3) for drawing out said cleaning composition (C) and an outlet (9) in communication with said pumping unit (4), said inlet (8) of the first conduit (7) being positioned substantially at a bottom (1a) of said bottle inside it.

9. The cleaning system according to any one of the preceding claims, wherein said spraying device comprises a second conduit (10) having an inlet (11) in fluid communication with the inside of the bottle (1) through the open neck (3) for drawing out said cleaning composition (C) and an outlet (12) in communication with said pumping unit (4), said inlet (11) of the second conduit (10) being positioned substantially at the open neck (3) of said bottle (1) inside it.

10. The cleaning system according to any one of claims 1 to 9, wherein the cleaning composition (C) comprises:
(A) 1-20% by weight, preferably 1-15% by weight, more preferably 1.5-10% by weight of a mixture of surfactants consisting of at least one anionic surfactant, at least one non-ionic surfactant and at least one amphoteric surfactant;
(B) water up to 100% by weight,
wherein the quantities of (A) and (B) are referred to the total weight of (A)+(B).

11. The cleaning system according to any one of claims 1 to 10, wherein in the cleaning composition (C):
- the non-ionic surfactant is selected in the group consisting of alkyl polyglucosides, alkyl polypentosides and mixtures thereof; and/or
- the amphoteric surfactant is selected in the group consisting of cocoamphodiacetates, cocoiminodipropionate, cocoamidopropylbetaine, cocoamidopropyl-dimethyl-hydroxysulphobetaine and mixtures thereof.

12. The cleaning system according to claim 10, wherein the anionic surfactant is selected in the group consisting of lauryl sulphate, lauryl ether sulphate, sulpholaurates, sulphoacetates, sulphosuccinates and mixtures thereof, preferably selected in the group consisting of sulpholaurates, sulphoacetates, sulphosuccinates and mixtures thereof, wherein more preferably said sulpholaurates, sulphoacetates and sulphosuccinates are non-ethoxylated.

13. The cleaning system according to claim 10, wherein the mixture of surfactants comprises a quantity ≥ 65% by weight, preferably 65-90% by weight, more preferably 70-85% by weight of the at least one anionic surfactant, wherein the quantity of anionic surfactant in the mixture of surfactants is referred to the total weight of the mixture (A) of surfactants.

14. A kit for preparing a cleaning system for personal hygiene comprising:
- a first component comprising a bottle (1) associated with the spraying device (2) as described in any one of claims 1 to 9; and
- a second component comprising the cleaning composition (C) as described in any one of claims 1, 10 to 13.

15. A method for obtaining a foam cleaner for personal hygiene comprising the step (i) of dispensing the cleaning composition (C) as described in any one of claims 1, 10-13 by actuating, preferably manually, the lever or trigger (5) of the spraying device (2) according to any one of claims 1 to 9.

16. A cleaning composition (C) as described in any one of claims 1, 10 to 13.

17. Use of the cleaning composition (C) according to claim 16 to obtain a foam cleaner for personal hygiene, said use comprising the dispensing of the cleaning composition (C) by means of the spraying device (2) as described in any one of claims 1 to 9.
